## Europäisches Patentamt
## European Patent Office
## Office européen des brevets

(19)

(11) Veröffentlichungsnummer : **0 037 393**
**B1**

(12) **EUROPÄISCHE PATENTSCHRIFT**

(45) Veröffentlichungstag der Patentschrift :
01.06.83

(51) Int. Cl.³ : **A 61 M 5/18**

(21) Anmeldenummer : **81890056.5**

(22) Anmeldetag : **01.04.81**

(54) **Vorrichtung zur Applikation eines Gewebeklebstoffes auf Basis von menschlichen oder tierischen Proteinen.**

(30) Priorität : **02.04.80 AT 1792/80**

(43) Veröffentlichungstag der Anmeldung :
**07.10.81 Patentblatt 81/40**

(45) Bekanntmachung des Hinweises auf die Patenterteilung : **01.06.83 Patentblatt 83/22**

(84) Benannte Vertragsstaaten :
**BE CH DE FR GB IT LI LU NL SE**

(56) Entgegenhaltungen :
**CH A 539 551**
**DE A 1 491 877**
**DE C 137 660**
**DE C 562 328**
**FR A 429 811**
**FR A 440 425**
**FR A 1 054 173**
**FR E 63 467**
**US A 3 016 897**
**US A 3 223 083**
**US A 3 467 096**
**US A 3 572 336**
**US A 4 109 653**

(73) Patentinhaber : **IMMUNO Aktiengesellschaft für che-
misch-medizinische Produkte**
**Industriestrasse 72**
**1220 Wien (AT)**

(72) Erfinder : **Redl, Heinz, Dipl.-Ing. Dr.**
**Klosterneuburgerstrasse 127/9**
**A-1220 Wien (AT)**
Erfinder : **Kriwetz, Gert, Dr.**
**Alte Poststrasse 419**
**A-8055 Graz (AT)**

(74) Vertreter : **Wolfram, Gustav, Dipl.-Ing.**
**Schwindgasse 7 P.O. Box 205**
**A-1041 Wien (AT)**

EP 0 037 393 B1

Vorrichtung zur Applikation eines Gewebeklebstoffes auf Basis von menschlichen oder tierischen Proteinen

Die Erfindung betrifft eine Vorrichtung zur Applikation eines Gewebeklebstoffes auf Basis von menschlichen oder tierischen Proteinen zum nahtlosen bzw. nahtunterstützenden Verbinden von menschlichen oder tierischen Gewebe- oder Organteilen, zur Wundversiegelung, Blutstillung u. dgl., welcher Gewebeklebstoff durch Zusammenbringen von Lösungen der Proteine und blutgerinnungsfördernder Gerinnungsfaktoren in situ gebildet wird, mit einer Halteeinrichtung für eine Mehrzahl von in Konussen endigenden Spritzenkörpern, vorteilhaft genormten Einwegspritzenkörpern aus Kunststoffmaterial, wobei die Konusse der einzelnen Spritzenkörper durch einen Sammelkopf mit Förderkanälen für die aus jedem der Konusse austretenden Komponenten des Gewebeklebstoffes verbunden sind.

Bei der Anwendung eines Gewebeklebstoffes der bezeichneten Art wird eine Faktor-XIII und Fibrinogen enthaltende Lösung mit einer Thrombin enthaltenden Lösung vermischt und auf die zu klebende bzw. zu schützende Wundstelle aufgebracht. Bekannte Applikationsweisen bestehen darin, die eine Lösung auf die Klebestelle aufzubringen und mit der anderen Lösung zu überschichten oder die beiden Lösungen in einem Mischgefäß oder auch auf einer Tüpfelplatte vorzumischen, in eine Spritze aufzuziehen und dann auf die zu klebende Stelle aufzutragen.

Beide Methoden haben Nachteile : Bei der ersten Methode tritt infolge unterschiedlicher Viskositäten der beiden Lösungen eine Verfestigung an der Oberfläche vorzeitig ein, bevor eine intensive Durchmischung stattgefunden hat.

Die Anwendung der zweiten Methode erfordert hohe Geschicklichkeit des medizinischen Personals, weil die Verfestigung des Klebers schon kurz nach dem Zusammenbringen der beiden Lösungen erfolgt. Es müssen innerhalb weniger Sekunden das Zusammenbringen der Komponenten, ihre Vermischung, Aufziehen in eine Spritze und das Auftragen auf die zu klebende Stelle erfolgen. Diese Methode ist daher auf nur wenige Anwendungsgebiete beschränkt.

Eine Vorrichtung der eingangs beschriebenen Art ist aus der US-PS 3,223,083 bekannt. Die Spritzenkörper werden von einer klammerartigen Halteeinrichtung umfaßt, und die Konusse der Spritzenkörper sind in ein Y-Stück eingesetz. In diesem Y-Stück werden die beiden Komponenten des Gewebeklebstoffes vermischt, wodurch bei einer kurzzeitigen Unterbrechung des Spritzvorganges eine Verfestigung des Gewebeklebstoffes im Y-Stück unvermeidlich ist. Es muß daher nach einer solchen Unterbrechung auch das Y-Stück ausgewechselt werden, was umständlich ist.

Die Erfindung bezweckt die Vermeidung der geschilderten Nachteile und Schwierigkeiten und stellt sich die Aufgabe, eine Vorrichtung der eingangs bezeichneten Art zu schaffen, mit der das Auftragen der Gewebeklebstoffkomponenten gleichzeitig unter ein-händischer Bedienung der Vorrichtung erfolgen kann, wobei eine zuverlässige Mischung erreicht und eine vorzeitige Verfestigung der Komponenten vor Erreichen der Wundstelle vermieden wird.

Diese Aufgabe wird erfindungsgemäß dadurch gelöst, daß die Förderkanäle bis zu ihren Mündungen vollständig voneinander getrennt sind und daß für die Kolben aller Spritzenkörper eine gemeinsame Betätigungseinrichtung vorgesehen ist. Für die Vorrichtung können genormte handelsübliche Einweg-Apparaturteile, z. B. Einwegspritzenkörper und -kolben sowie genormte Kanülen verwendet werden, die in steriler Verpackung im Handel erhältlich und im ärztlichen Instrumentarium vorhanden sind.

Vorteilhaft ist auf den Sammelkopf eine Mischkanüle aufsetzbar. Zwecks besserer Handhabung ist die gemeinsame Betätigungseinrichtung für die Spritzenkolben an einer die Haltevorrichtung durchsetzenden Führungsstange geführt.

Zweckmäßig sind die Mündungen der Förderkanäle eng benachbart an der Stirnseite eines Aufsteckkonus des Sammelkopfes angeordnet.

Vorteilhaft liegen die Mündungen der Förderkanäle des Sammelkopfes im Bereich von Austrittsöffnungen von im Winkel zueinander geführten Zuführungskanälen für sterile Gase.

Nach einer weiteren vorteilhaften Ausführungsform sind die Achsen der Austrittsöffnungen der Zuführungskanäle in einem spitzen Winkel zueinander gerichtet und liegt der Winkelscheitel im Abstand, vorzugsweise im Abstand von 10-20 cm vor dem Mündungen der Förderkanäle.

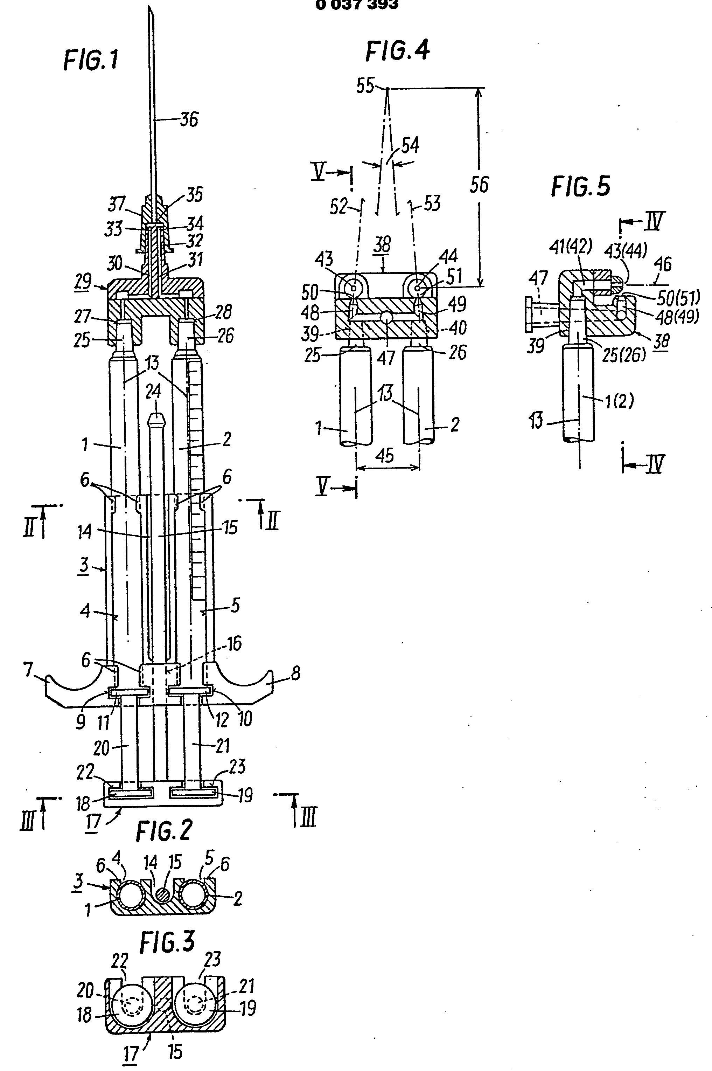

Die erfindungsgemäße Vorrichtung ist nachfolgend anhand der Zeichnung an zwei Ausführungsformen näher erläutert, wobei Fig. 1 eine teilweise geschnittene Seitenansicht der Vorrichtung, Fig. 2 einen Schnitt gemäß der Linie II-II und Fig. 3 einen Schnitt gemäß der Linie III-III der Fig. 1 einer ersten Ausführungsform zeigen. In den Fig. 4 und 5 ist eine zweite Ausführungsform teilweise im Schnitt dargestellt, wobei Fig. 4 einen Schnitt gemäß der Linie IV-IV der Fig. 5 und Fig. 5 einen Schnitt gemäß der Linie V-V der Fig. 4 veranschaulichen.

Mit 1 und 2 sind zwei Spritzenkörper bezeichnet, von denen einer zur Aufnahme einer Thrombin enthaltenden Lösung und der zweite zur Aufnahme einer Faktor-XIII und Fibrinogen enthaltenden Lösung dient. Die Spritzenkörper 1, 2 sind zweckmäßig als genormte Einwegspritzenkörper aus Kunststoff ausgebildet. Sie sind gemeinsam in eine Halteeinrichtung 3 eingesetzt. Die Halteeinrichtung 3 weist zwei U-förmig gestaltete Rinnen 4, 5 auf, die jeweils an ihren Enden mit den größten Querschnitt jeder Rinne übergreifenden Noppen 6 ausgestaltet sind, sodaß die Spritzenkörper 1, 2, die von oben in die Rinnen 4, 5 eingesetzt werden, einrasten und durch die Noppen 6 in den Rinnen festgehalten werden. An dem zwei seitliche Fingergriffe 7, 8 aufweisenden Ende der Halteeinrichtung sind U-förmig gestaltete Erweiterungen 9, 10 der Rinnen vorgesehen, in die die Flanschenden 11, 12 der Spritzenkörper 1, 2 ragen, sodaß die Spritzenkörper 1, 2 auch in Richtung ihrer jeweiligen Längsachse 13 in der Halteeinrichtung 3 fixiert sind.

Mittig zwischen den beiden U-förmigen Rinnen 4, 5 ist eine Ausnehmung 14 für eine Führungsstange 15 vorgesehen, die im Bereich des die Fingergriffe 7, 8 aufweisenden Endes der Halteeinrichtung 3 in eine dieses Ende durchsetzende Bohrung 16 übergeht. Die Führungsstange 15 ist mit einer Betätigungseinrichtung 17, in die die Daumenaufsätze 18, 19 der Kolben 20, 21 der Spritzenkörper 1, 2 eingesetzt sind, einstückig verbunden. Die Daumenaufsätze 18, 19 der Kolben 20, 21 sind jeweils in eine U-förmig ausgebildete Ausnehmung 22, 23 der Betätigungseinrichtung 17 eingesetzt. Die Führungsstange 15 weist an ihrem vorderen Ende eine Verdickung 24 auf, sodaß sie nicht aus der Bohrung 16 der Halteeinrichtung 3 herausgleiten kann.

Die beiden Konusse 25, 26 der Spritzenkörper ragen in Einsteck-Konusse 27, 28 eines Sammelkopfes 29 und sind durch diesen Sammelkopf miteinander verbunden. Innerhalb des Sammelkopfes 29 führt von jedem Einsteck-Konus 27, 28 ein eigener Förderkanal 30, 31 bis in einen am Sammelkopf vorgesehenen Aufsteck-Konuskopf 32. Die Mündungen 33, 34 der Förderkanäle 30, 31 sind eng benachbart an der Stirnseite 35 dieses Aufsteck-Konuskopfes vorgesehen. Eine Mischkanüle 36, die an ihrem hinteren Ende mit einem genormten Einsteck-Konus 37 versehen ist, läßt sich auf den Aufsteck-Konuskopf 32 aufschieben.

Durch Druck mit dem Daumen auf die Betätigungseinrichtung 17 treten aus beiden Spritzenkörpern 1, 2 gleichzeitig vorbestimmte Volumina aus. Infolge des geringen Abstandes der Mündungen 33, 34 der Förderkanäle 30, 31 kommt es zum sofortigen Kontakt der Lösungen, wobei der Vermischungseffekt optimal ist. Die Auftragung mit einer Spritzenfüllung kann sowohl intremittierend als auch kontinuierlich erfolgen. Verbrauchte Spritzenkörper 1, 2 und die Mischkanüle 36 können rasch ausgetauscht werden und die gesamte Vorrichtung kann gegebenenfalls problemlos sterilisiert werden. Die Ein-Handbedienung und die automatische Dosierung der beiden Lösungen im gewünschten Verhältnis stellen eine wesentliche Arbeitserleichterung dar. Wird eine Mischkanüle 36 verwendet, so ist die Vermischung besonders intensiv, wodurch Klebungen von höchster Festigkeit erreicht werden können. Wird die Applikation des Gewebeklebers unterbrochen, so muß die Mischkanüle 36 abgenommen und durch eine neue ersetzt werden. Der Sammelkopf 29 hingegen kann weiter verwendet werden. Die beschriebene Vorrichtung gestattet eine Anwendung des Gewebeklebstoffes auf nahezu allen Gebieten.

Die in den Fig. 4 und 5 dargestellte Ausführungsform ermöglicht das Aufsprühen des Gewebeklebers auf eine zu verklebende oder zu versiegelnde Fläche. Bei dieser Ausführungsform ist der Sammelkopf als Sprühkopf 38 ausgebildet. Er weist, von den Einsteck-Konussen 39, 40 ausgehend, zwei voneinander getrennte Förderkanäle 41, 42 auf, deren Mündungen 43, 44 in einem etwa dem Abstand 45 der Spritzenkörper 1, 2 entsprechendem Abstand voneinander angeordnet sind. Die Austrittsrichtung 46 der Lösungen aus den Mündungen 43, 44 ist etwa rechtwinkelig zur Längsrichtung 13 der Spritzenkörper 1, 2.

Zusätzlich zu den Förderkanälen 41, 42 weist der Sammel-bzw. Sprühkopf 38 einen Zuführkanal 47 für ein steriles Gas, beispielsweise Druckluft, auf, der sich innerhalb des Sammelkopfes in zwei Äste 48, 49 teilt. Die Austrittsöffnungen 50, 51 dieser beiden Zuführungskanäle 48, 49 sind im Bereich der Mündungen 43, 44 der Förderkanäle 41, 42 angeordnet. Die Achsen 52, 53 der Austrittsöffnungen 50, 51 sind etwa rechtwinkelig zur Austrittsrichtung 46 der Lösungen gerichtet, wobei die Achsen 52, 53 der Austrittsöffnungen in einem spitzen Winkel 54 zueinander gerichtet sind und der Winkelscheitel 55 in einem Abstand 56 von etwa 10 bis 20 cm vor den Mündungen der Förderkanäle liegt.

Bei dieser Ausführungsform erfolgt die Dosierung der Lösungen ebenfalls mit Hilfe der Betätigungseinrichtung, die gleich gestaltet ist wie die Betätigungseinrichtung der in Fig. 1 dargestellten Ausführungsform. Die Applikation des Gewebeklebers kann ohne Wechsel der Sprüheinrichtung 38 unterbrochen werden. Die beiden Sprühkegel vereinigen sich in etwa 10 bis 20 cm Entfernung und bilden auf der zu verklebenden bzw. zu versiegelnden Fläche rasch einen dünnen gleichmäßigen Gewebeklebstofffilm. Die Durchmischung der Komponenten ist auch bei dieser Ausführungsform als optimal anzusehen. Zusätzlich bietet diese Ausführungsform noch den Vorteil, daß vor der eigentlichen Applikation die Klebestelle getrocknet, d.h. Körperflüssigkeit mittels Druckluft weggeblasen werden kann, wodurch eine bessere Haftung des Klebers erreicht wird. Durch die gute Dosierbarkeit des Gewebeklebers ist das Arbeiten mit dieser Ausführungsform der Vorrichtung besonders sparsam.

Der Sprühkopf läßt eine Anwendung des Gewebeklebstoffes für größere Flächen zu. Besonders bevorzugte Anwendungsgebiete sind die Blutstillung, Wundversiegelung, Verbrennungen, Hauttransplantationen und das Aussprühen von Körperhöhlen.

**Ansprüche**

1. Vorrichtung zur Applikation eines Gewebeklebstoffes auf Basis von menschlichen oder tierischen Proteinen zum nahtlosen bzw. nahtunterstützenden Verbinden von menschlichen oder tierischen Gewebe- oder Organteilen, zur Wundversiegelung, Blutstillung u. dgl., welcher Gewebeklebstoff durch Zusammenbringen von Lösungen der Proteine und blutgerinnungsfördernder Gerinnungsfaktoren in situ gebildet wird, mit einer Halteeinrichtung (3) für eine Mehrzahl von in Konussen (25, 26) endigenden Spritzenkörpern (1, 2), vorteilhaft genormten Einwegspritzenkörpern aus Kunststoffmaterial, wobei die Konusse (25, 26) der einzelnen Spritzenkörper (1, 2) durch einen Sammelkopf (29, 38) mit Förderkanälen (30, 31, 41, 42) für die aus jedem der Konusse austretenden Komponenten des Gewebeklebstoffes verbunden sind, dadurch gekennzeichnet, daß die Förderkanäle (30, 31, 41, 42) bis zu ihren Mündungen (33, 34 ; 43, 44) vollständig voneinander getrennt sind und daß für die Kolben (20, 21) aller Spritzenkörper (1, 2) eine gemeinsame Betätigungseinrichtung (17) vorgesehen ist.

2. Vorrichtung nach Anspruch 1, dadurch gekennzeichnet, daß auf den Sammelkopf eine Mischkanüle (36) aufsetzbar ist.

3. Vorrichtung nach Anspruch 1 oder 2, dadurch gekennzeichnet, daß die gemeinsame Betätigungseinrichtung (17) für die Spritzenkolben an einer die Haltevorrichtung (3) durchsetzenden Führungsstange (15) geführt ist.

4. Vorrichtung nach Anspruch 1, dadurch gekennzeichnet, daß die Mündungen (33, 34) der Förderkanäle (30, 31) eng benachbart an der Stirnseite (35) eines Aufsteckkonus (32) des Sammelkopfes (29) angeordnet sind.

5. Vorrichtung nach den Ansprüchen 1 bis 4, dadurch gekennzeichnet, daß die Mündungen (43, 44) der Förderkanäle (41, 42) des Sammelkopfes (29, 38) im Bereich von Austrittsöffnungen (50, 51) von im Winkel zueinander geführten Zuführungskanälen (48, 49) für sterile Gase liegen (Fig. 4, 5).

6. Vorrichtung nach Anspruch 5, dadurch gekennzeichnet, daß die Achsen (52, 53) der Austrittsöffnungen (50, 51) der Zuführungskanäle (48, 49) in einem spitzen Winkel (54) zueinander gerichtet sind und der Winkelscheitel (55) im Abstand (56), vorzugsweise im Abstand von 10-20 cm vor den Mündungen (43, 44) der Förderkanäle (41, 42) liegt.

## Claims

1. Arrangement for applying a tissue adhesive based on human or animal proteins to seamlessly or seam-supportingly connect human or animal tissue or organ parts, to seal wounds, to stop bleedings and the like, which tissue adhesive is formed in situ by bringing together solutions of the proteins and of clot-promoting coagulation factors, comprising a holding means (3) for a plurality of syringe bodies (1, 2) ending in coni (25, 26), preferably standardized one-way syringe bodies of synthetic material, the coni (25, 26) of the individual syringe bodies (1, 2) being connected by a collecting head (29, 38) including conveying channels (30, 31, 41, 42) for the components of the tissue adhesive emerging from each of the coni, characterised in that the conveying channels (30, 31, 41, 42) are completely separated from one another as far as to their mouths (33, 34 ; 43, 44), and that a common actuation means (17) is provided for the plungers (20, 21) of all the syringe bodies (1, 2).

2. Arrangement according to claim 1, characterised in that a mixing needle (36) is slippable onto the collecting head.

3. Arrangement according to claim 1 or 2, characterised in that the common actuation means (17) for the syringe plungers are guided along a guiding rod (15) penetrating the holding means (3).

4. Arrangement according to claim 1, characterised in that the mounths (33, 34) of the conveying channels (30, 31) are arranged on the front side (35) of slip-on cone (32) of the collecting heat (29) in a closely neighbouring manner.

5. Arrangement according to claims 1 to 4, characterised in that the mouths (43, 44) of the conveying channels (41, 42) of the collecting head (29, 38) are located in the region of outlet openings (50, 51) of supplying channels (48, 49) for sterile gases guided at an angle to each other (Figs. 4, 5).

6. Arrangement according to claim 5, characterised in that the axes (52, 53) of the outlet openings (50, 51) of the supplying channels (48, 49) are directed to each other at an acute angle (54), and that the angle vertex (55) lies at a distance (56), preferably at a distance of 10 to 20 cm, in front of the mouths (43, 44) of the conveying channels (41, 42).

## Revendications

1. Dispositif pour l'application d'une colle pour tissus à base de protéines humaines ou animales pour l'union sans suture ou de soutien de suture de parties d'organes ou de tissus humains ou animaux, pour l'isolement étanche de plaies, l'hémostase et analogues, laquelle colle pour tissus est formée in situ par la mise en contact de solutions des protéines et des facteurs de coagulation qui favorisent la coagulation du sang, avec une installation de support (3) pour une multiplicité de corps de seringue (1, 2) se terminant par des cônes (25, 26) avantageusement des corps de seringue normalisés à n'utiliser qu'une seule fois en une matière plastique, où les cônes (25, 26) des corps de seringue individuels (1, 2) sont reliés par l'intermédiaire d'une tête collectrice (29, 38) comportant des canaux d'alimentation (30, 31, 41, 42) pour les composants de la colle pour tissus sortant de chacun des cônes, caractérisé en ce que des canaux d'alimentation (30, 31, 41, 42) sont mutuellement totalement séparés jusqu'à leurs sorties (33, 34, 43, 44) et en ce qu'un dispositif de commande ou d'actionnement (17) commun est prévu pour les pistons (20, 21) de tous les corps de seringue (1, 2).

2. Dispositif suivant la revendication 1, caractérisé en ce qu'une aiguille de mélange (36) peut être adaptée sur la tête collectrice.

3. Dispositif suivant l'une quelconque des revendications 1 et 2, caractérisé en ce que le dispositif de commande commun (17) pour les pistons des seringues est guidé par une tige de guidage (15) qui traverse le dispositif de support (3).

4. Dispositif suivant la revendication 1, caractérisé en ce que les sorties (33, 34) des canaux d'alimentation (30, 31) sont logées à proximité étroite l'une de l'autre du côté frontal (35) d'un cône de raccordement (32) de la tête collectrice (29).

5. Dispositif suivant l'une quelconque des revendications 1 à 4, caractérisé en ce que les sorties (43, 44) des canaux d'alimentation (41, 42) de la tête collectrice (29, 38) se trouvent à portée ou dans la zone d'ouvertures d'éjection (50, 51) de canaux d'amenée (48, 49) de gaz stériles, dirigés angulairement l'un vers l'autre (fig. 4, 5).

6. Dispositif suivant la revendication 5, caractérisé en ce que les axes (52, 53) des ouvertures d'éjection (50, 51) des canaux d'amenée (48, 49) sont dirigés l'un vers l'autre selon un angle aigu (54) et en ce que le sommet de l'angle (55) se situe à distance (56), avantageusement à une distance de 10 à 20 cm, des sorties (43, 44) des canaux d'alimentation (41, 42).

FIG.1

FIG.4

FIG.5

FIG.2

FIG.3